# EUROPEAN PATENT APPLICATION

(11) **EP 4 674 445 A1**
(43) Date of publication of application: **07.01.2026**
(21) Application number: 24770762.3
(22) Date of filing: 08.03.2024
(51) Int. Cl.: A61L 29/08, A61L 31/10

(54) **MEDICAL TOOL AND METHOD FOR MANUFACTURING MEDICAL TOOL**

(30) Priority: 10.03.2023 JP 2023037345
(71) Applicant: TERUMO Kabushiki Kaisha, Tokyo 151-0072 (JP)
(72) Inventor: WATANABE, Nozomu, Fujinomiya-shi, Shizuoka 418-0015 (JP); HOSONO, Hiroki, Ashigarakami-gun, Kanagawa 259-0151 (JP); UENO, Yuki, Ashigarakami-gun, Kanagawa 259-0151 (JP)
(74) Representative: Casalonga
(86) International application number: PCT/JP2024/009010
(87) International publication number: WO 2024/190656

(57) **Abstract**

The present invention provides a method for manufacturing a medical device having a surface lubricating layer exhibiting excellent durability. The present invention relates to a method for manufacturing a medical device including a base layer and a surface lubricating layer supported on at least a part of the base layer, the method including: preparing a coating liquid including a block copolymer having a structural unit (A) derived from a reactive monomer having an epoxy group and a structural unit (B) derived from a hydrophilic monomer, at least one polymer material selected from polyvinyl chloride and polyurethane, and a solvent; and applying the coating liquid onto the base layer.

## Description

### Technical Field

The present invention relates to a medical device and a method for manufacturing a medical device.

### Background Art

Medical devices inserted into a living body such as a catheter, a guide wire, and an indwelling needle are required to exhibit an excellent lubricating property in order to reduce damage to tissues such as a blood vessel and improve operability of an operating surgeon. For this reason, a method for coating a base layer surface with a hydrophilic polymer having the lubricating property has been developed and put to practical use. In such a medical device, elution and peeling of the hydrophilic polymer from the surface of the base layer is a problem in terms of safety and maintenance of operability. Therefore, coating with a hydrophilic polymer is required not only to have the excellent lubricating property but also to have durability against loads such as wear and abrasion.

From such a viewpoint, JP H8-33704 A discloses a medical device in which a water-soluble or water-swellable polymer is dissolved in a solvent in which a base material of the medical device swells to produce a polymer solution, the base material of the medical device is immersed in the polymer solution to swell, and the polymer is crosslinked or polymerized on the surface of the base material to form a surface lubricating layer on the surface of the base material. According to the technique disclosed in JP H8-33704, a surface lubricating layer exhibiting a relatively good lubricating property can be immobilized to a base material.

### Summary of Invention

JP H8-33704 A discloses that it is preferable to use, as a water-soluble or water-swellable polymer, a block copolymer composed of a hydrophilic moiety exhibiting lubricating property and a moiety having an epoxy group. When such a block copolymer is used, the epoxy group of the block copolymer can be crosslinked by a heating operation, and a surface lubricating layer that is relatively difficult to peel off can be formed. However, a good lubricating property and excellent durability are in a trade-off relationship, and a technique for achieving both the good lubricating property and excellent durability is required.

In particular, miniaturization and diameter reduction of medical devices in recent years are remarkable, and medical procedures for approaching a medical device to a lesion site through a living body lumen such as a narrow blood vessel having higher flexibility in a living body are spreading. In addition, as the medical procedure becomes complicated, the operation of the medical device may take a long time. Therefore, in order to retain good operability of the medical device even at a complicated lesion site, there is a demand for a technique for further improving the lubrication retaining property (durability) of the medical device surface (surface lubricating layer) as compared with the prior art. More specifically, there is a demand for a device having excellent sliding durability and capable of retaining a high lubricating property even when sliding of a surface (surface lubricating layer) of a medical device is repeated.

Therefore, there is a demand for a technology capable of improving durability (In particular, sliding durability) of a medical device and supporting a medical procedure that becomes complicated and advanced.

The present invention has been made in view of the above circumstances, and an object thereof is to provide a method for manufacturing a medical device having a surface lubricating layer that exhibits the excellent durability (a lubrication retaining property) (in particular, sliding durability).

The present inventors have intensively studied to solve the above problems. As a result, the present inventors have found that the above object can be achieved by combining a polymer material having an electron-withdrawing property with a hydrophilic block copolymer having an epoxy group, and have completed the present invention.

The above object can be achieved by the present invention having the following configuration, and the present invention includes the following aspects and forms.

One aspect of the present invention is
1. a method for manufacturing a medical device including a base layer and a surface lubricating layer supported on at least a part of the base layer, the method including: preparing a coating liquid including a block copolymer having a structural unit (A) derived from a reactive monomer having an epoxy group and a structural unit (B) derived from a hydrophilic monomer, at least one polymer material selected from polyvinyl chloride and polyurethane, and a solvent; and applying the coating liquid onto the base layer.
2. In the manufacture method described in the above 1., the content of the polymer material in the coating liquid is preferably smaller than a content of the block copolymer in the coating liquid.
3. In the manufacture method according to 1. or 2. above, the coating liquid preferably includes the polymer material in an amount of 0.001% by mass or more and less than 12% by mass.
4. In the manufacture method according to any one of 1. to 3. above, the reactive monomer having an epoxy group preferably includes at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.
5. In the manufacture method according to any one of 1. to 4. above, the hydrophilic monomer preferably includes at least one selected from the group consisting of N,N-dimethylacrylamide, acrylamide, 2-hydroxyethyl methacrylate, and N-vinylpyrrolidone.

Another aspect of the present invention is
6. a medical device including: a base layer; and a surface lubricating layer supported on at least a part of the base layer, wherein
   the surface lubricating layer includes a block copolymer having a structural unit (A) derived from a reactive monomer having an epoxy group and a structural unit (B) derived from a hydrophilic monomer, and at least one polymer material selected from polyvinyl chloride and polyurethane, and
   in the surface lubricating layer, the block copolymer and the polymer material are interpenetrated with each other.
7. In the medical device of 6. above, the amount of the polymer material present in the surface lubricating layer is preferably smaller than the amount of the block copolymer present in the surface lubricating layer.
8. In the medical device according to the above 6. or 7. above, the reactive monomer having an epoxy group preferably includes at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.
9. In the medical device according to any one of 6. to 8. above, the hydrophilic monomer preferably includes at least one selected from the group consisting of N,N-dimethylacrylamide, acrylamide, 2-hydroxyethyl methacrylate, and N-vinylpyrrolidone.

### Brief Description of Drawings

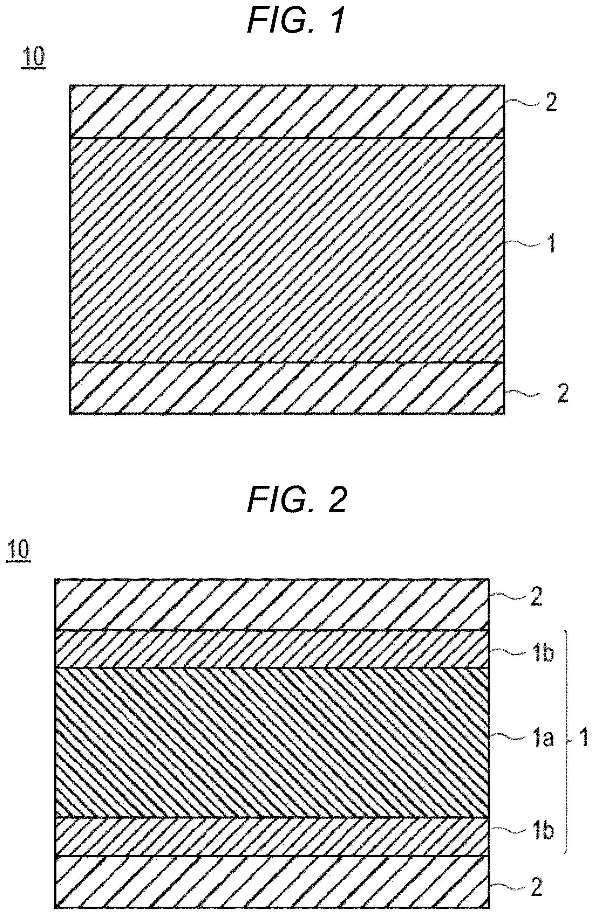

Fig. 1 is a partial cross-sectional view schematically illustrating a lamination structure of a surface of a representative embodiment of a medical device according to the present invention. In Fig. 1, reference numeral 10 indicates a medical device; reference numeral 1 indicates a base layer; reference numeral 2 indicates a lubricating layer, respectively.
Fig. 2 is a partial cross-sectional view schematically illustrating a different structure example of the lamination structure of the surface as an application example of the embodiment of Fig. 1. In Fig. 2, reference numeral 10 indicates a medical device; reference numeral 1a indicates a base layer core portion; reference numeral 1b: base material surface layer; reference numeral 2 indicates a lubricating layer, respectively.

### Description of Embodiments

A method for manufacturing a medical device according to an aspect of the present invention is a method for manufacturing a medical device including a base layer and a surface lubricating layer supported on at least a part of the base layer, the method including: preparing a coating liquid including a block copolymer having a structural unit (A) derived from a reactive monomer having an epoxy group and a structural unit (B) derived from a hydrophilic monomer, at least one polymer material selected from polyvinyl chloride and polyurethane, and a solvent; and applying the coating liquid onto the base layer. According to the present invention, it is possible to manufacture a medical device having a surface lubricating layer that exhibits the excellent durability (lubrication retaining property) (in particular, sliding durability).

In the present specification, the structural unit (A) derived from a reactive monomer having an epoxy group is also simply referred to as a "structural unit (A) according to the present invention" or "structural unit (A)". In the present specification, the structural unit (B) derived from a hydrophilic monomer is also simply referred to as a "structural unit (B) according to the present invention" or "structural unit (B)". In the present specification, the block copolymer having the structural units (A) and (B) is also simply referred to as a "block copolymer according to the present invention" or "block copolymer".

In the present specification, at least one polymer material selected from polyvinyl chloride and polyurethane is also simply referred to as a "polymer material according to the present invention" or "polymer material".

In the present specification, when a structural unit is defined to be "derived" from a certain monomer, it means that the structural unit is a structural unit generated by cleavage of one bond of a polymerizable unsaturated double bond of the corresponding monomer.

In the present specification, the term "(meth)acryl" represents both acryl and methacryl. Therefore, for example, the term "(meth)acrylic acid" encompasses both acrylic acid and methacrylic acid. Similarly, the term "(meth)acryloyl" refers to both acryloyl and methacryloyl. Therefore, for example, the term "(meth)acryloyl group" refers to both an acryloyl group and a methacryloyl group. Further similarly, the term "(meth)acrylate" encompasses both acrylates and methacrylates. For example, the term "alkoxyalkyl (meth)acrylate" encompasses both an alkoxyalkyl acrylate and an alkoxyalkyl methacrylate.

In the present specification, a range from "X to Y" includes X and Y, and indicates "X or more and Y or less". In addition, "X and/or Y" includes each of X and Y and all combinations of one or more, and specifically means at least one of X and Y, and includes X alone, Y alone, and a combination of X and Y.

Unless otherwise specified, operations and measurements of physical properties and the like are performed under the conditions of room temperature (20 to 25°C)/relative humidity 40 to 50%RH.

In the present invention, a coating liquid including a block copolymer having the structural unit (A) derived from a reactive monomer having an epoxy group and the structural unit (B) derived from a hydrophilic monomer, at least one (polymer material) of polyvinyl chloride and polyurethane, and a solvent is prepared, and the coating liquid is applied onto the base layer.

That is, the present invention is characterized by forming a surface lubricating layer using a block copolymer and at least one (polymer material) of polyvinyl chloride and polyurethane. The polymer material according to the present invention has the electron-withdrawing property. Therefore, when the polymer material comes into contact with the block copolymer, the electrons of the epoxy group (crosslinked part) are peeled off, the bond is broken, and the epoxy group is ring-opened. When the epoxy group is ring-opened, crosslinking (bonding) between the block copolymers proceeds, and the film strength of the surface lubricating layer increases. When the base layer is a resin material, the ring-opened epoxy group also causes crosslinking (bonding) between the block copolymer and the base layer. Since the ring-opening reaction of the epoxy group is electron transfer between molecules, the polymer material and the block copolymer (hydrophilic polymer molecule) need to approach each other until the electron orbitals of the molecules overlap. However, in the coating liquid, since the solvent is interposed between the polymer material and the block copolymer, the polymer material and the block copolymer are not in close contact with each other but exist apart from each other. Therefore, the polymer material does not attract electrons of the epoxy group (crosslinked part), and ring-opening (crosslinking reaction) of the epoxy group hardly proceeds or does not proceed at all in the coating liquid. Since the ring-opening of the epoxy group of the block copolymer does not proceed, the coating liquid does not have high viscosity or gelation, and can maintain the form of the solution. Therefore, the coating liquid can be uniformly applied onto the base layer. On the other hand, when the coating liquid is applied onto the base layer, and then the solvent is removed from the coating film of the coating liquid by heating and drying or the like, the polymer material comes into contact with the block copolymer to attract electrons (electron orbitals of molecules overlap), and ring-opening (crosslinking reaction) of the epoxy group is initiated and promoted. According to the method of the present invention, the coating liquid can be uniformly applied onto the base layer, and the ring-opening (crosslinking reaction) of the epoxy group uniformly and densely proceeds on the surface of the base layer, so that the film strength of the surface lubricating layer can be increased, and the block copolymer can be more firmly immobilized to the surface of the base layer. As a result, it is presumed that a surface lubricating layer (strong cover layer) having high film strength is formed, the strong cover layer can be favorably maintained even after sliding against a living body lumen such as a narrow blood vessel having higher flexibility, and a high lubricating property (surface lubricating property) can be retained for a longer period of time (that is, an excellent surface lubricating property can be retained, and sliding durability can be improved). In particular, when heat treatment is applied to the coating film of the coating liquid (heat energy is applied), electron transfer more actively occurs due to expansion and contraction of molecules by heat energy, and ring-opening (crosslinking reaction) of the epoxy group is further promoted, so that the durability of the surface lubricating layer can be further improved. Note that the above mechanism is an inference and does not limit the technical scope of the invention.

Therefore, the medical device manufactured according to the method of the present invention has the excellent durability (surface lubrication retaining property and sliding durability). In addition, the medical device manufactured according to the method of the present invention also has the excellent lubricating property.

Hereinafter, embodiments of the present invention will be described. Note that the present invention is not limited only to the following embodiments, and various modifications can be made within the scope of claims. In addition, the embodiments described in the present specification can be arbitrarily combined with each other to form another embodiment. The dimensional ratios in the drawings are exaggerated for convenience of description and may be different from actual ratios. In addition, in a case where embodiments of the present invention are described with reference to the drawings, the same elements are denoted by the same reference numerals in the description of the drawings, and redundant description will be omitted.

Throughout the present specification, expression of a singular should be understood to include also a concept of a plural thereof unless otherwise stated. Thus, an article of a singular (for example, "a", "an", and "the" in English) should be understood to include also a concept of a plural thereof unless otherwise stated. In addition, terms used in the present specification should be understood to be used in a sense commonly used in the art unless otherwise stated. Therefore, unless otherwise defined, all technical and scientific terms used in the present specification have the same meanings as commonly understood by a person skilled in the art to which the present invention belongs. In a case of contradiction, priority is given to the present specification (including definitions).

### <Method for manufacturing medical device>

A method for manufacturing a medical device according to the present invention includes: preparing a coating liquid including a block copolymer having a structural unit (A) derived from a reactive monomer having an epoxy group and a structural unit (B) derived from a hydrophilic monomer, at least one polymer material selected from polyvinyl chloride and polyurethane, and a solvent ((I) preparation step); applying the coating liquid onto the base layer ((II) coating step). As described above, by using a solution including a specific polymer material together with the block copolymer as a coating liquid, a medical device having a surface lubricating layer (cover layer) exhibiting the excellent durability can be obtained.

After the step (II), steps such as a step of performing drying and/or heat treatment ((III) drying/heat treatment step) and a step of washing ((IV) washing step) may be further performed as necessary. Among them, it is preferable to further perform at least a (III) drying/heat treatment step after the step (II). In the method of the present invention, the polymer material is stably held in the surface lubricating layer. In addition, the epoxy group of the block copolymer is ring-opened without requiring addition of an acid or a base. Therefore, in the method of the present invention, it is not particularly necessary to perform the (IV) washing step, which is advantageous in mass production.

### (I) Preparation step

In this step, a coating liquid including a block copolymer, a polymer material, and a solvent is prepared. Here, in this step, a coating liquid may be prepared by mixing a block copolymer, a polymer material, and a solvent to prepare a coating liquid. Alternatively, a coating liquid including the block copolymer, the polymer material, and the solvent may be purchased, and the coating liquid may be used.

Hereinafter, preferred aspects of preparing a coating liquid by mixing a block copolymer, a polymer material, and a solvent to prepare the coating liquid will be described in detail.

### (Block copolymer)

In the present invention, the block copolymer forms a surface lubricating layer supported on at least a part of the base layer in a state of being interpenetrated with the polymer material. That is, in the medical device obtained by the method according to the present invention, the surface lubricating layer includes a block copolymer and a polymer material. The term "supporting" means a state in which the surface lubricating layer is immobilized in a state of not being easily released from the surface of the base layer, and includes not only a form in which the entire surface of the base layer is completely covered with the surface lubricating layer, but also a form in which only a part of the surface of the base layer is covered with the surface lubricating layer, that is, a form in which the surface lubricating layer is attached to only a part of the surface of the base layer.

The block copolymer according to the present invention has the structural unit (A) derived from a reactive monomer having an epoxy group and the structural unit (B) derived from a hydrophilic monomer.

The reactive monomer having an epoxy group constituting the block copolymer has an epoxy group as a reactive group. The epoxy group is ring-opened to promote crosslinking (bonding) between the block copolymers, and the film strength of the surface lubricating layer is increased by introducing the structural unit (A) derived from such a reactive monomer into the block copolymer. When the base layer is a resin material, the ring-opened epoxy group also causes crosslinking (bonding) between the block copolymer and the base layer.

The reactive monomer constituting the block copolymer is not particularly limited as long as it has an epoxy group (preferably a glycidyl group), and a known compound can be used. Among them, the reactive monomer having an epoxy group preferably contains at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl methacrylate, and allyl glycidyl ether because crosslinking or polymerization of the block copolymer can be easily controlled.

Among them, glycidyl (meth)acrylate is more preferable, and glycidyl methacrylate is particularly preferable in consideration of further promoting the crosslinking reaction, ease of manufacture, and the like.

One type of the reactive monomer may be used independently or two or more types may be used in combination. That is, the reactive site derived from the reactive monomer may be a homopolymer type composed of one reactive monomer alone or a copolymer type composed of two or more reactive monomers. In the case of using two or more kinds, the form of the reactive site may be a block copolymer (block form) or a random copolymer (random form).

That is, in a preferred embodiment of the present invention, the reactive monomer having an epoxy group includes at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether. In a more preferred embodiment of the present invention, the reactive monomer having an epoxy group is at least one of glycidyl acrylate and glycidyl methacrylate. In a particularly preferred form of the invention, the reactive monomer having an epoxy group is glycidyl methacrylate.

The hydrophilic monomer constituting the block copolymer has a swelling property upon contact with a body fluid (for example, blood, urine) or an aqueous solvent, and therefore imparts a lubricating property (surface lubricating property) to the medical device. Therefore, by introducing the structural unit (B) derived from a hydrophilic monomer into the block copolymer, the lubricating property (surface lubricating property) of the medical device is improved, and friction when the medical device comes into contact with a lumen wall such as a blood vessel wall can be reduced.

The hydrophilic monomer constituting the block copolymer is not particularly limited as long as it has the above characteristics, and a known compound can be used. Examples thereof include acrylamide and derivatives thereof, vinylpyrrolidone, acrylic acid and methacrylic acid and derivatives thereof, polyethylene glycol acrylate and derivatives thereof, a monomer having a sugar and a phospholipid in a side chains and a water-soluble monomer such as maleic anhydride. More specifically, examples thereof include acrylic acid, methacrylic acid, N-methylacrylamide, N,N-dimethylacrylamide (DMAA), acrylamide, acryloylmorpholine, N,N-dimethylaminoethyl acrylate, N-vinylpyrrolidone, 2-methacryloyloxyethyl phosphorylcholine, 2-methacryloyloxyethyl-D-glycoside, 2-methacryloyloxyethyl-D-mannoside, vinyl methyl ether, 2-hydroxyethyl (meth)acrylate, 4-hydroxybutyl (meth)acrylate, 2-hydroxypropyl (meth)acrylate, 2-hydroxybutyl (meth)acrylate, 6-hydroxyhexyl (meth)acrylate, 1,4-cyclohexanedimethanol mono(meth)acrylate, 1-chloro-2-hydroxypropyl (meth)acrylate, diethylene ethylene glycol mono(meth)acrylate, 1,6-hexanediol mono(meth)acrylate, pentaerythritol tri(meth)acrylate, dipentaerythritol penta(meth)acrylate, neopentyl glycol mono(meth)acrylate, trimethylolpropane di(meth)acrylate, trimethylolethane di(meth)acrylate, 2-hydroxy-3-phenyloxypropyl (meth)acrylate, 4-hydroxycyclohexyl (meth)acrylate, 2-hydroxy-3-phenyloxy (meth)acrylate, 4-hydroxycyclohexyl(meth)acrylate, cyclohexanedimethanol mono(meth)acrylate, poly(ethylene glycol) methyl ether acrylate, and poly(ethylene glycol) methyl ether methacrylate. From the viewpoint of imparting an excellent lubricating property, ease of synthesis, and operability, the hydrophilic monomer preferably includes at least one selected from the group consisting of N,N-dimethylacrylamide, acrylamide, 2-hydroxyethyl methacrylate, and N-vinylpyrrolidone, and more preferably is at least one kind selected from the group consisting of N,N-dimethylacrylamide, acrylamide, and 2-hydroxyethyl methacrylate.

Among them, from the viewpoint of the excellent lubricating property, the hydrophilic monomer is particularly preferably N,N-dimethylacrylamide.

One type of the hydrophilic monomer may be used independently or two or more types may be used in combination. That is, the hydrophilic moiety derived from the hydrophilic monomer may be a homopolymer type composed of one hydrophilic monomer or a copolymer type composed of two or more hydrophilic monomers. In the case of using two or more kinds, the form of the hydrophilic moiety may be a block copolymer (block form) or a random copolymer (random form).

That is, in a preferred embodiment of the present invention, the hydrophilic monomer includes at least one selected from the group consisting of N,N-dimethylacrylamide, acrylamide, 2-hydroxyethyl methacrylate, and N-vinylpyrrolidone. In a more preferred embodiment of the present invention, the hydrophilic monomer is at least one kind selected from the group consisting of N,N-dimethylacrylamide, acrylamide, and 2-hydroxyethyl methacrylate. In a particularly preferred form of the invention, the hydrophilic monomer is N,N-dimethylacrylamide.

The block copolymer has the structural unit (A) and the structural unit (B). Here, the ratio between the structural unit (A) and the structural unit (B) is not particularly limited as long as the above effect is obtained. In consideration of a good lubricating property, a lubrication retaining property, strength of the cover layer, bondability to the base layer, and the like, the ratio of the structural unit (A) to the structural unit (B) (molar ratio of the structural unit (A) : the structural unit (B)) is preferably 1 : 2 to 1 : 100, more preferably 1 : 2 to 1 : 50, still more preferably 1 : 5 to 1 : 50, and particularly preferably 1 : 10 to 1 : 30. Within such a range, the surface lubricating layer can sufficiently exhibit the lubricating property by the structural unit (B), and can exhibit sufficient cover layer strength, bondability to the base layer (in the case of the resin material), and durability by the structural unit (A). The molar ratio of the structural unit (A) : the structural unit (B) can be controlled by adjusting the charging ratio (molar ratio) of each monomer in the manufacture stage of the block copolymer. Therefore, the charging ratio (molar ratio) between the reactive monomer having an epoxy group and the hydrophilic monomer in the manufacture stage of the block copolymer is preferably 1 : 2 to 1 : 100, more preferably 1 : 2 to 1 : 50, still more preferably 1 : 5 to 1 : 50, and particularly preferably 1 : 10 to 1 : 30. The molar ratio of the structural unit (A) : the structural unit (B) can be confirmed, for example, by performing NMR measurement (¹H-NMR measurement, ¹³C-NMR measurement, etc.) on the copolymer.

The block copolymer according to the present invention essentially includes the structural unit (A) and the structural unit (B), but may have other structural units in addition to these structural units. When the block copolymer has other structural units, examples of the other structural units include adipic acid, glutaric acid, triethylene glycol, and tripropylene glycol. The monomers constituting other structural units may be used singly, or may be used in combination of two or more thereof. That is, the other structural unit may be a homopolymer type composed of one kind of structural unit alone or a copolymer type composed of two or more kinds of structural units. When two or more monomers constituting the other structural unit are used, the segment constituted by the monomers may be in the form of a block copolymer, a random copolymer, or an alternating copolymer.

When the block copolymer according to the present invention has other structural units, the content of the other structural units is preferably more than 0 mol% and 5 mol% or less with respect to all the structural units constituting the block copolymer. That is, in the block copolymer according to the present invention, when the total of all structural units constituting the block copolymer is 100 mol%, the total of the contents of the structural unit (A) and the structural unit (B) is preferably 95 mol% or more (upper limit: less than 100 mol%).

More preferably, the block copolymer according to the present invention is substantially composed of the structural unit (A) and the structural unit (B) (the content of other structural units = more than 0 mol% and less than 5 mol%). In this form, the block copolymer according to the present invention can achieve durability by the structural unit (A) and the lubricating property (surface lubricating property) by the structural unit (B) in a well-balanced manner. Preferably, the block copolymer according to the present invention does not include the other structural unit (content of other structural unit = 0 mol%).

The composition of each structural unit (structural units (A) and (B) and other structural units) can be measured by a known method. For example, the composition (molar ratio) of the structural unit can be measured by measuring the integral ratio of the intensity of each signal in the ¹H-NMR spectrum of the block copolymer solution.

In an embodiment of the present invention, the block copolymer according to the present invention is substantially composed of a structural unit (A) derived from at least one reactive monomer selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether, and a structural unit (B) derived from at least one hydrophilic monomer selected from the group consisting of N,N-dimethylacrylamide, acrylamide, 2-hydroxyethyl methacrylate, and N-vinylpyrrolidone, or is composed only of the above.

In an embodiment of the present invention, the block copolymer according to the present invention is substantially composed of a structural unit (A) derived from a reactive monomer of at least one of glycidyl acrylate and glycidyl methacrylate, and a structural unit (B) derived from at least one hydrophilic monomer selected from the group consisting of N,N-dimethylacrylamide, acrylamide, and 2-hydroxyethyl methacrylate, or is composed only of the above.

In an embodiment of the present invention, the block copolymer according to the present invention is substantially composed of a structural unit (A) derived from glycidyl methacrylate (reactive monomer having an epoxy group) and a structural unit (B) derived from N,N-dimethylacrylamide (hydrophilic monomer), or is composed only of the above.

The weight average molecular weight of the block copolymer is preferably 10,000 to 10,000,000 from the viewpoint of solubility. The weight average molecular weight of the block copolymer is more preferably 100,000 to 5,000,000 from the viewpoint of ease of preparation of a coating liquid. In the present specification, as the "weight average molecular weight", a value measured by gel permeation chromatography (Gel Permeation Chromatography, GPC) using polystyrene as a reference material is adopted.

The method for manufacturing the block copolymer is not particularly limited, and the block copolymer can be produced by applying a conventionally known polymerization method such as a living radical polymerization method, a polymerization method using a macro initiator, or a polycondensation method. Among them, a living radical polymerization method or a polymerization method using a macro initiator is preferably used because it is easy to control the molecular weight and molecular weight distribution of the structural unit (moiety) derived from a reactive monomer and the structural unit (moiety) derived from a hydrophilic monomer. The living radical polymerization method is not particularly limited, but for example, methods described in JP H11-263819 A, JP 2002-145971 A, JP 2006-316169 A, and the like, an atom transfer radical polymerization (ATRP) method, and the like can be applied in the same manner or appropriately modified. In addition, in a polymerization method using a macro initiator, for example, a macro initiator having a reactive site having a reactive functional group and a radical polymerizable group such as a peroxide group is produced, and then a monomer for forming a hydrophilic moiety is polymerized with the macro initiator, whereby a block copolymer having a hydrophilic moiety and a reactive site can be produced.

The block copolymer after polymerization is preferably purified by a general purification method such as a reprecipitation method, a dialysis method, an ultrafiltration method, or an extraction method.

### (Polymer material)

In the present invention, the polymer material includes at least one selected from polyvinyl chloride and polyurethane. The polymer material has the electron-withdrawing property. Therefore, when the polymer material and the block copolymer come into contact with each other, electrons of the epoxy group (crosslinked part) of the block copolymer are peeled off to induce ring-opening of the epoxy group. When the epoxy group is ring-opened, crosslinking (bonding) between the block copolymers proceeds, and the film strength of the surface lubricating layer increases. When the base layer is a resin material, the ring-opened epoxy group also causes crosslinking (bonding) between the block copolymer and the base layer. Therefore, in the medical device obtained by the method according to the present invention, durability (sliding durability) can be further improved, and the shape can be favorably maintained even after sliding. In addition, polymer materials (Polyvinyl chloride, polyurethane) are suitable in terms of safety since they are already used as medical materials.

The promotion of crosslinking of the block copolymer as described above may also occur by the addition of an acid or a base to the coating liquid. However, in such a case, crosslinking or polymerization between the block copolymers rapidly proceeds in the coating liquid due to an acid or a base present in the coating liquid, so that the viscosity of the coating liquid becomes high (or gelled), and it may be difficult to uniformly apply the coating liquid. On the other hand, in the polymer material, since the contact with the block copolymer is inhibited by the solvent in the coating liquid, crosslinking or polymerization of the block copolymer is less likely to proceed in the coating liquid. In addition, the polymer material promotes the crosslinking reaction when the solvent is removed by heating and drying or the like and the polymer material comes into contact with the block copolymer. Therefore, it is possible to suppress an increase in viscosity (gelation) of the coating liquid due to crosslinking of the block copolymer in the coating liquid as described above. Therefore, according to the method of the present invention, the coating liquid can be uniformly applied onto the base layer. In addition, when an acid or a base is added to the coating liquid, it is necessary to perform an operation such as a washing step of removing the acid or the base remaining in the surface lubricating layer or a neutralization step of neutralizing the acid or the base remaining in the surface lubricating layer after forming the surface lubricating layer. On the other hand, in the method of the present invention, since the above-described process is not separately required, the work efficiency can be enhanced.

In the present specification, the "electron-withdrawing property" refers to a property of easily attracting an electron from the bonded atom side as compared with a hydrogen atom.

The polymer material according to the present invention includes at least one of polyvinyl chloride and polyurethane. Among them, polyvinyl chloride has a higher electron-withdrawing property so that crosslinking by the structural unit (A) of the block copolymer (particularly during heat treatment) can be further promoted (durability can be further improved). In addition, polyvinyl chloride has higher solvent solubility (excellent coatability). Therefore, the polymer material preferably includes at least polyvinyl chloride, and more preferably includes only polyvinyl chloride.

The weight average molecular weight (Mw) of the polymer material is 1,000 or more. From the viewpoint of solubility, the weight average molecular weight of the polymer material is preferably 500,000 or less. From the viewpoint of promoting crosslinking and stability, the weight average molecular weight of the polymer material is preferably 10,000 or more. As an example, the weight average molecular weight (Mw) of the polymer material is 1,000 to 10,000,000, preferably 10,000 to 500,000.

### (Preparation of coating liquid)

A coating liquid is prepared using the block copolymer, the polymer material, and the solvent. Here, since the polymer material is stable in the coating liquid, it is preferable from the viewpoint of safety and simplicity of operation. In addition, in the coating liquid, a solvent is interposed between the polymer material and the block copolymer, and the polymer material and the block copolymer are not in close contact with each other but exist separately from each other. On the other hand, the ring-opening (crosslinking reaction) of the epoxy group is started and progressed by approaching the block copolymer until the electron orbitals of the molecules of the block copolymer and the polymer material overlap each other and exchanging electrons. For this reason, in the coating liquid, the ring-opening (crosslinking reaction) of the epoxy group hardly proceeds or does not proceed at all, and the viscosity of the coating liquid hardly changes or does not change at all. Therefore, workability is excellent.

The order of addition and the method of addition of the block copolymer, the polymer material, and the solvent are not particularly limited. The above components may be added collectively or separately, stepwise or continuously. The mixing method is not particularly limited, and a known method can be used. Examples of the method for preparing the coating liquid include a method of sequentially adding a polymer material and a block copolymer to a solvent, a method of sequentially adding a block copolymer and a polymer material to a solvent, and a method of collectively adding a polymer material and a block copolymer to a solvent. Preferably, the polymer material and the block copolymer are sequentially added into the solvent, or the block copolymer and the polymer material are sequentially added into the solvent. In addition, if necessary, the above addition may be performed while stirring. Alternatively, the mixed solution may be stirred after the addition.

The solvent used for preparing the coating liquid is not particularly limited as long as it can dissolve the block copolymer and the polymer material (and other components, if used.), and is appropriately selected according to the type of the block copolymer and the polymer material (and other components, if used). Alcohol-based solvents such as methanol, ethanol, isopropyl alcohol and butanol from the viewpoint of high solubility; organic solvents such as dichloromethane, chloroform, carbon tetrachloride, tetrahydrofuran (THF), dimethyl sulfoxide, N,N-dimethylformamide (DMF), dioxane, and benzene are preferably used. These solvents may be used singly or in mixture of two or more types thereof (in a form of a mixed solvent).

The concentration of the block copolymer in the coating liquid is not particularly limited. From the viewpoint of further improving coatability, lubricating property and durability of the surface lubricating layer, the concentration of the block copolymer in the coating liquid is preferably 0.5 to 20% by mass, more preferably 1 to 15% by mass, and particularly preferably 3 to 10% by mass. When the concentration of the block copolymer is within the above range, the lubricating property and durability of the resulting surface lubricating layer can be sufficiently exhibited. In addition, a uniform surface lubricating layer having a desired thickness can be easily obtained by one coating, and the viscosity of the solution falls within an appropriate range, which is preferable in terms of operability (ease of coating, for example) and production efficiency. However, even if it is out of the above range, it can be sufficiently used as long as it does not affect the operation and effect of the present invention.

The concentration of the polymer material in the coating liquid is also not particularly limited. From the viewpoint of the lubricating property (sliding property) of the surface lubricating layer, it is preferable that the addition amount of the polymer material is small. On the other hand, from the viewpoint of durability (sliding durability) of the surface lubricating layer, the polymer material is preferably added to some extent. From the above viewpoint, the concentration of the polymer material in the coating liquid is preferably 0.001% by mass or more and less than 12% by mass, more preferably 0.005% by mass or more and 4.0% by mass or less, still more preferably 0.01% by mass or more and less than 0.5% by mass, and particularly preferably 0.01 to 0.1% by mass. When the concentration of the polymer material is within the above range, the crosslinking of the block copolymer is not allowed to proceed excessively while sufficiently securing the lubricating property (sliding property) of the surface lubricating layer (crosslinking can be moderately promoted). Therefore, the lubricating property and durability of the resulting surface lubricating layer can be sufficiently exhibited. However, even if it is out of the above range, it can be sufficiently used as long as it does not affect the operation and effect of the present invention.

Furthermore, the mixing ratio of the block copolymer and the polymer material in the coating liquid is such that the content of the polymer material in the coating liquid is preferably equal to or less than the content of the block copolymer in the coating liquid from the viewpoint of further improvement in durability (sliding durability), coatability, the lubricating property of the surface lubricating layer, and the like, and is more preferably less than the content of the block copolymer in the coating liquid from the viewpoint of further improvement in durability (sliding durability). More specifically, the mixing ratio of the block copolymer and the polymer material (block copolymer : polymer material (mass ratio)) in the coating liquid is, for example, 100 : 0.01 or more and 100 : 100 or less, preferably 100 : 0.02 or more and less than 100 : 100, more preferably 100 : 0.1 or more and less than 100 : 90, still more preferably more than 100 : 0.1 and less than 100 : 70, still more preferably more than 100 : 0.15 and less than 100 : 50, particularly preferably 100 : 0.2 or more and less than 100 : 10, and most preferably more than 100 : 0.20 and less than 100 : 3.0. When the mass ratio of the block copolymer and the polymer material is within the above range, the durability and lubricating property of the resulting surface lubricating layer can be sufficiently exhibited.

### (Other components)

The coating liquid for forming the surface lubricating layer may include other components in addition to the block copolymer, the polymer material, and the solvent. For example, in a case where the medical device is intended to be inserted into a body cavity or a lumen such as a catheter, the other component is not particularly limited, and examples thereof include agents (physiologically active substances) such as an anticancer drug, an immunosuppressive agent, an antibiotic, an antirheumatic drug, an antithrombotic drug, an HMG-CoA reductase inhibitor, an ACE inhibitor, a calcium antagonist, an antilipemic agent, an integrin inhibitor, an antiallergic agent, an antioxidant, a GPIIb/IIIa antagonist, a retinoid, a flavonoid, a carotenoid, a lipid improver, a DNA synthesis inhibitor, a tyrosine kinase inhibitor, an antiplatelet drug, a vascular smooth muscle proliferation inhibitor, an anti-inflammatory drug, a biological material, an interferon, and an NO production promoter. Here, the amount of other components to be added is not particularly limited, and the amounts usually used are similarly applied. Finally, the addition amount of other components is appropriately selected in consideration of the severity of the applied disease, the weight of the patient, and the like.

### (II) Coating step

In this step, the coating liquid prepared in the (I) preparation step is applied onto the base layer to form a coating film (coating layer) on the base layer.

The base layer may be made of any material, and examples thereof include a metal material, a polymer material (resin material), and ceramics.

Among the materials constituting the base layer, the metal material is not particularly limited, and metal materials generally used for medical devices such as a catheter, a guide wire, and an indwelling needle are used. Specific examples thereof include various stainless steels such as SUS304, SUS314, SUS316, SUS316L, SUS420J2, and SUS630, gold, platinum, silver, copper, nickel, cobalt, titanium, iron, aluminum, tin, or various alloys such as a nickel-titanium alloy, a nickel-cobalt alloy, a cobalt-chromium alloy, and a zinc-tungsten alloy. These may be used singly or in combination of two or more kinds thereof. As the metal material, an optimal metal material may be appropriately selected as a base layer of a catheter, a guide wire, an indwelling needle, or the like which is used.

In addition, among the materials constituting the base layer, the polymer material (resin material or elastomer material) is not particularly limited, and a polymer material generally used for medical devices such as a catheter, an introducer, a guide wire, and an indwelling needle is used. Specific examples thereof include a polyamide resin, polyolefin resins such as a polyethylene resin and a polypropylene resin, a modified polyolefin resin, a cyclic polyolefin resin, an epoxy resin, a polyurethane resin, a diallyl phthalate resin (allyl resin), a polycarbonate resin, a fluororesin, an amino resin (a urea resin, a melamine resin, a benzoguanamine resin), a polyethylene terephthalate resin and polyester resins such as a polybutylene terephthalate resin, a styrol resin, an acrylic resin, a polyacetal resin, a vinyl acetate resin, a phenol resin, a vinyl chloride resin, a silicone resin (silicon resin), a polyether resin and a polyimide resin.

Thermoplastic elastomers such as a polyurethane elastomer, a polyester elastomer, and a polyamide elastomer (nylon elastomer) can also be used as the material of the base layer.

These polymer materials may be used singly, or may be used as a mixture of two or more thereof or as a copolymer of two or more monomers constituting any of the resin or elastomer. Among them, as the polymer material, a polyethylene resin, a polyurethane resin, a polyethylene terephthalate resin, a polyamide resin, and a polyamide elastomer are preferable, and a polyamide resin and a polyamide elastomer are more preferable. A carboxy group or an amino group as an end group included in the polyamide resin or the polyamide elastomer may undergo a crosslinking reaction with an epoxy group in the block copolymer. In addition, these polymer materials (particularly, polyamide resin and polyamide elastomer) are relatively soft, and it can be said that the block copolymer constituting the surface lubricating layer easily penetrates into these polymer materials. Therefore, the bondability between the polymer material (particularly, the polyamide resin and the polyamide elastomer) and the block copolymer is enhanced, and a surface lubricating layer having more excellent durability can be formed. As the polymer material, a polymer material optimal for a base layer of a catheter, a guide wire, an indwelling needle, or the like that is used may be appropriately selected.

The shape of the base layer is not particularly limited, and is appropriately selected according to a use mode such as a sheet shape, a linear shape (wire), a rod shape, or a tubular shape.

The method for applying the coating liquid to the surface of the base layer is not particularly limited, and conventionally known methods such as a coating/printing method, an immersion method (dipping method, dip coating method), a spraying method, a spin coating method, a mixed solution impregnation sponge coating method, a bar coating method, a die coating method, a reverse coating method, a comma coating method, a gravure coating method, and a doctor knife method can be applied. Among them, an immersion method (dipping method, dip coating method) is preferably used.

When a surface lubricating layer is formed on a thin and narrow inner surface of a catheter, a guide wire, an injection needle, or the like, the base layer may be immersed in a coating liquid to depressurize the inside of the system to defoam. The solution can be quickly permeated into the thin and narrow inner surface to promote the formation of the surface lubricating layer by reducing the pressure to defoam.

When the surface lubricating layer is formed only on a part of the base layer, the surface lubricating layer can be formed on a desired surface portion of the base layer by immersing only a part of the base layer in a coating liquid and coating the part of the base layer with the coating liquid.

When it is difficult to immerse only a part of the base layer in the coating liquid, a surface portion of the base layer on which the surface lubricating layer does not need to be formed is protected (covered or the like) in advance with an appropriate member or material that can be attached/detached (donned/doffed), the base layer is immersed in the coating liquid to coat the base layer with the coating liquid, then a protective member (material) of the surface portion of the base layer on which the surface lubricating layer does not need to be formed is removed, and then the protective member is reacted by heat treatment or the like, whereby the surface lubricating layer can be formed on a desired surface portion of the base layer. However, the present invention is not limited to these forming methods, and the surface lubricating layer can be formed by appropriately using a conventionally known method. For example, when it is difficult to immerse only a part of the base layer in the coating liquid, another coating method (for example, a method for applying the coating liquid to a predetermined surface portion of the medical device using a spray device or a coating device such as a bar coater, a die coater, a reverse coater, a comma coater, a gravure coater, a spray coater, or a doctor knife) may be applied instead of the immersion method.

Note that in a case where both the outer surface and the inner surface of the cylindrical tool need to have a surface lubricating layer due to the structure of the medical device, an immersion method (dipping method) is preferably used because both the outer surface and the inner surface can be coated at a time.

The coating amount of the coating liquid is preferably such an amount that the thickness (dry film thickness) of the resulting coating (surface lubricating layer) is 0.1 to 10 µm, more preferably such an amount that the thickness is 0.5 to 5 µm, and still more preferably such an amount that the thickness is 1 to 3 µm. When the coating amount is such that the thickness of the coating (surface lubricating layer) is 0.1 µm or more, the durability of the obtained coating (surface lubricating layer) can be sufficiently achieved. Furthermore, when the coating amount is such that the thickness of the coating (surface lubricating layer) is 10 um or less, the surface of the coating (surface lubricating layer) is less likely to be sticky, and handling at the time of manufacturing becomes easier.

### (III) Drying/heat treatment step

In the method for manufacturing a medical device according to the present invention, if necessary, a coating film (coating layer) may be formed by coating a coating liquid onto the base layer in the (II) coating step, and then a drying step and/or a heat treatment step may be performed. For the purpose of removing the solvent and forming a strong surface lubricating layer, it is preferable to perform a drying step and/or a heat treatment step, and it is more preferable to perform the drying step and the heat treatment step.

Here, although the "drying treatment" and the "heat treatment" are not strictly distinguished from each other, for convenience of description, the "drying treatment" refers to holding the base layer to which the coating liquid is applied at a temperature (20 to 30°C) or lower around room temperature, and the "heat treatment" refers to holding the base layer at a temperature exceeding the temperature (20 to 30°C) around room temperature.

The conditions at the time of drying or heat treatment are not particularly limited as long as the conditions allow the surface lubricating layer including the block copolymer to be formed on the base layer.

The temperature of the drying or heat treatment is not particularly limited, but is preferably 10 to 200°C. That is, it is more preferable to maintain the coating layer at 10 to 200°C after coating the block copolymer solution onto the base layer (after forming the coating layer). Crosslinking or polymerization of the block copolymer is effectively promoted by maintaining the temperature at such a temperature, and a strong cover layer (surface lubricating layer) is formed. Accordingly, it is possible to retain high lubricating property (surface lubricating property) for a longer period of time. In addition, it is possible to suppress excessive progress of the crosslinking or polymerization by maintaining the temperature at such a temperature. Therefore, it is possible to prevent reduction in the swelling property due to excessive curing of the surface lubricating layer, thereby, it is possible to retain good lubricating property (surface lubricating property).

Furthermore, it is more preferable to maintain the coating layer at 200°C or lower, still more preferable to maintain the coating layer at 20 to 150°C, and most preferable to maintain the coating layer at 50 to 130°C after applying the block copolymer solution onto the base layer (after forming the coating layer). By maintaining at such a temperature, the crosslinking reaction is further promoted, and a surface lubricating layer having higher durability can be formed. In addition, the surface lubricating layer can exhibit excellent lubricating property. In particular, when the temperature is 150°C or lower, excessive crosslinking or polymerization of the block copolymer can be suppressed. In addition, it is possible to prevent or suppress a decrease in hydrophilicity of the structural unit (B) of the block copolymer. Therefore, it is possible to suppress a decrease in the swelling property due to excessive curing of the surface lubricating layer, and it is possible to more easily control the lubricating property. The temperature may be changed during the drying or heating treatment.

Furthermore, the time for drying or heat treatment is not particularly limited but is preferably 30 minutes to 30 hours, more preferably, 1 to 25 hours, and particularly preferably, 1 to 12 hours. Crosslinking or polymerization in the block copolymer is effectively promoted by setting such a time, and a strong cover layer (surface lubricating layer) is formed. Accordingly, it is possible to retain high lubricating property (surface lubricating property) for a longer period of time. In addition, the above time range prevents excessive progress of crosslinking or polymerization. Therefore, it is possible to prevent reduction in the swelling property due to excessive curing of the surface lubricating layer, thereby, it is possible to retain good lubricating property (surface lubricating property). In particular, by setting the heat treatment time to 25 hours (particularly 15 hours or 12 hours) or less, a decrease in hydrophilicity of the structural unit (B) of the block copolymer can be prevented or suppressed, and lubricating property can be more easily controlled. When the base layer is composed of the polymer material (resin material or elastomer material), the time for drying or heat treatment may be shorter, for example, about 15 minutes to 5 hours, 30 minutes to 3 hours. When the time is short as described above, damage to the hydrophilic group in the structural unit (B) (reduction in hydrophilicity of the structural unit (B) of the block copolymer) can be further suppressed, so that the surface lubricating layer can exhibit better lubricating property. In addition, there is an advantage that even a polymer material that is easily deformed or plasticized by heat can be used as a base layer. Therefore, according to the present invention, the selectivity of the material is further widened, and medical devices for various applications can be manufactured. In addition, since it is possible to form the surface lubricating layer having excellent durability at a low temperature, it is preferable from the viewpoint of energy cost at the time of manufacturing a medical device.

In this step, from the viewpoint of particularly effectively (efficiently) promoting crosslinking or polymerization of the block copolymer, it is preferable to further perform a heat treatment after performing a drying treatment. As described above, through the drying and the heat treatment, the heat treatment is further performed in a state where the solvent is distilled off (that is, in a state where the block copolymer and the polymer material are likely to come into contact with each other), so that the effect of promoting crosslinking or polymerization of the block copolymer by the polymer material is further improved. In addition, since the heat treatment can be performed in a shorter time, even a polymer material that is easily deformed or plasticized by heat can be used as the base layer.

The conditions (temperature, time, etc.) of the drying and heat treatment at this time are also not particularly limited, but from the viewpoint of efficiently manufacturing a medical device, in a case where the base layer is formed of a polymer material, it is preferable to perform a drying treatment of maintaining at 10 to 30°C for 15 minutes to 5 hours and then perform a heat treatment of maintaining at 40 to 200°C for 30 minutes to 10 hours. From the same viewpoint, it is more preferable to perform a drying treatment of maintaining at 15 to 30°C for 30 minutes to 3 hours and then a heat treatment of maintaining at 45 to 150°C for 1 to 6 hours, and it is particularly preferable to perform a drying treatment of maintaining at 20 to 25°C for 30 minutes to 1.5 hours and then a heat treatment of maintaining at 50 to 130°C for 1 to 3 hours. From the same viewpoint, when the base layer is composed of a metal material, it is preferable to perform a drying treatment of maintaining at 10 to 30°C for 15 minutes to 5 hours and then a heat treatment of maintaining at 40 to 200°C for 5 to 20 hours. From the same viewpoint, it is more preferable to perform a drying treatment of maintaining at 15 to 30°C for 30 minutes to 3 hours and then a heat treatment of maintaining at 80 to 150°C for 8 to 18 hours, and it is particularly preferable to perform a drying treatment of maintaining at 20 to 25°C for 30 minutes to 1.5 hours and then a heat treatment of maintaining at 100 to 130°C for 10 to 15 hours. Under such conditions, a medical device including the surface lubricating layer having excellent durability can be manufactured. After the heat treatment, a drying treatment may be further performed.

Under the above conditions (temperature, time, etc.), a strong surface lubricating layer (cover layer) can be supported on the surface of the base layer. In addition, depending on the type of the base layer, a crosslinking reaction via the epoxy group in the block copolymer in the surface lubricating layer occurs, and a high-strength surface lubricating layer that is not easily peeled off from the base layer can be formed. Therefore, peeling of the surface lubricating layer from the base layer can be effectively suppressed and prevented by the drying/heat treatment step.

In addition, the pressure condition during drying is not limited at all, and the drying may be performed under normal pressure (atmospheric pressure), or may be performed under pressure or reduced pressure.

As the drying or heating means (device), for example, an oven, a vacuum dryer, or the like can be used, but in the case of natural drying, the drying means (device) is particularly unnecessary.

### <Medical device>

Through the (I) preparation step and the (II) coating step described above, and the (III) drying/heat treatment step performed as necessary, a medical device having a surface lubricating layer (cover layer) exhibiting excellent durability can be manufactured.

That is, according to the method of the present invention, after forming the coating layer including the block copolymer and the polymer material on the surface of the base layer, the epoxy group is crosslinked to form a strong surface lubricating layer that is not easily peeled off from the base layer. In addition, in the medical device obtained by the method according to the present invention, since the surface lubricating layer formed of the block copolymer is formed on the surface, excellent lubricating property and durability (lubrication retaining property) can be exhibited.

A medical device manufactured by the method according to the present invention has a structure in which a coating liquid including a block copolymer for forming a surface lubricating layer and a polymer material is applied onto a base layer, so that the surface lubricating layer is supported on the base layer. In the coating liquid, the polymer material and the block copolymer are dissolved in a solvent (the polymer material and the block copolymer are uniformly mixed in the coating liquid). Therefore, the block copolymer and the polymer material are interpenetrated with each other in the surface lubricating layer.

Therefore, according to another aspect of the present invention, there is provided a medical device including: a base layer; and a surface lubricating layer supported on at least a part of the base layer, wherein the surface lubricating layer includes a block copolymer having a structural unit (A) derived from a reactive monomer having an epoxy group and a structural unit (B) derived from a hydrophilic monomer, and at least one polymer material selected from polyvinyl chloride and polyurethane, and in the surface lubricating layer, the block copolymer and the polymer material are interpenetrated with each other. In this aspect, the terms such as the block copolymer and the polymer material are the same as those described in <Method for manufacturing medical device> above, and thus the description thereof is omitted here. As described above, the content of the polymer material in the coating liquid is preferably smaller than the content of the block copolymer in the coating liquid. Therefore, the amount of the polymer material present in the surface lubricating layer is preferably smaller than the amount of the block copolymer present in the surface lubricating layer. At this time, the preferred abundance ratio (content ratio) of the block copolymer and the polymer material present in the surface lubricating layer is the same as the preferred mixing ratio of the block copolymer and the polymer material in the coating liquid in the above (polymer material).

Hereinafter, preferred embodiments of a medical device manufactured by the method according to the present invention will be described with reference to the accompanying drawings.

Fig. 1 is a partial cross-sectional view schematically illustrating a lamination structure of a surface of a representative embodiment of a medical device (In the present specification, it is also abbreviated as "medical device") manufactured by a method according to the present invention. Fig. 2 is a partial cross-sectional view schematically illustrating a different structure example of the laminated structure on the surface as an application example of the present embodiment. Note that reference numerals in Fig. 1 and Fig. 2 indicate the following. Reference numeral 1 indicates a base layer; reference numeral 1a indicates a base layer core portion; reference numeral 1b indicates a base material surface layer; reference numeral 2 indicates a surface lubricating layer; and reference numeral 10 indicates a medical device manufactured by the method according to the invention, respectively.

As shown in Fig. 1 and Fig. 2, a medical device 10 of the present embodiment includes a base layer 1 and a surface lubricating layer 2 provided on at least a part of the base layer 1 and including a block copolymer (in the drawing, an example in which the film is provided on the entire surface (whole surface) of the base layer 1 in the drawing is shown).

In Fig. 1 and Fig. 2, the surface lubricating layer 2 is formed on both surfaces of the base layer 1, but the present invention is not limited to the above embodiment, and may be any form such as a form in which it is formed on one surface of the base layer 1; and a form in which it is formed on a part of one surface or both surfaces of the base layer 1.

Hereinafter, the medical device will be described in detail for each component.

### (Base layer (base material))

The base layer used in the present embodiment may be composed of any material, and the material is not particularly limited. Specific examples of the material constituting the base layer 1 include a metal material, a polymer material, and ceramics. Specific examples of the material constituting the base layer 1 are as described in the (II) coating step.

Here, in the base layer 1, the entire base layer 1 may be made of any of the above materials. The base layer 1 may be a multilayer structure formed by laminating different materials in multiple layers, a structure in which members formed of different materials are connected for each part of a medical device, or the like. Alternatively, as shown in Fig. 2, the base layer core portion 1a may have a structure in which the base material surface layer 1b is formed by covering the surface of the base layer core portion 1a formed of any of the above materials with any other of the above materials by an appropriate method. Examples of the latter case include: the layer in which the surface of the base layer core portion 1a formed of a resin material or the like is covered with a metal material by an appropriate method (conventionally known methods such as plating, metal vapor deposition, and sputtering) to form the base material surface layer 1b; and the layer in which the surface of a base layer core portion 1a formed of a hard reinforcing material such as a metal material or a ceramic material is covered with a polymer material that is softer than the reinforcing material such as a metal material by an appropriate method (conventionally known methods such as immersion (dipping), spraying, and coating/printing), or the reinforcing material that forms the base layer core portion 1a and the polymer material are combined to form a substrate surface layer 1b. Further, the base layer core portion 1a may have a multilayer structure formed by laminating different materials in multiple layers, a structure in which members formed of different materials are connected for each part of the medical device, or the like. Another middle layer (not shown) may be further formed between the base layer core portion 1a and the base material surface layer 1b. Furthermore, the base material surface layer 1b may also have a multilayer structure formed by laminating different materials in multiple layers, a structure in which members formed of different materials are connected for each part of the medical device, or the like.

### (Surface Lubricating Layer (cover layer))

The surface lubricating layer is supported on at least a part of the base layer 1. Here, the reason why the surface lubricating layer 2 is supported on at least a part of the surface of the base layer 1 is that, in a medical device such as a catheter, a guide wire, or an indwelling needle to be used, it is not always necessary that all surfaces (the entire surface) of these medical devices have the lubricating property in a wet state, and it is sufficient that the surface lubricating layer is supported only on a surface portion (which may be a part or all) where the surface is required to have the lubricating property in a wet state. Therefore, as described above, the surface lubricating layer includes: a form formed so as to cover the entire both surfaces of the base layer as shown in Fig. 1 and Fig. 2; a form formed so as to cover only entire one surface of base layer; a form formed so as to cover a part of both surfaces of the base layer in the same or different form; a form formed so as to cover a part of one surface of base layer, and the like.

### <Application of medical device>

The medical device manufactured by the method of the present invention is a device used in contact with a body fluid, blood, or the like, and has a surface having the lubricating property in an aqueous liquid such as a body fluid or physiological saline, and can improve operability and reduce damage to mucosal tissue. Specific examples thereof include a catheter, a guide wire, and an indwelling needle used in a blood vessel, and the following medical devices are also shown.

(a) Catheters inserted into or indwelled in a digestive organ orally or nasally, such as a gastric tube catheter, a feeding catheter, or a feeding tube.
(b) Catheters inserted or indwelled into or in the airway or trachea orally or nasally, such as an oxygen catheter, an oxygen cannula, a tube or cuff of an endotracheal tube, a tube or cuff of a tracheostomy tube, and an intratracheal aspiration catheter.
(c) Catheters inserted into or indwelled in the urethra or ureter, such as a catheter or a balloon of a urethral tube, a urinary catheter, and a urinary balloon catheter.
(d) Catheters inserted into or indwelled in various body cavities, organs, and tissues such as suction catheters, drainage catheters, and rectal catheters.
(e) Catheters inserted into or indwelled in a blood vessel, such as an indwelling needle, an IVH catheter, a thermolysion catheter, an angiographic catheter, a vasodilator catheter, a dilator, or an introducer, or a guide wire, a stylet, or the like for these catheters.
(f) Artificial trachea, artificial bronchus, etc.
(g) Medical devices for extracorporeal circulation treatment (an artificial lung, an artificial heart, an artificial kidney, etc.) and circuits thereof.

### Examples

The effects of the present invention will be described with reference to the following Examples and Comparative Examples. Note that the technical scope of the present invention is not limited only to the following Examples. Note that, in the following Examples, unless otherwise specified, an operation was performed at room temperature (25°C). In addition, unless otherwise specified, "%" and "parts" mean "% by mass" and "parts by mass", respectively.

### Synthesis Example 1

The following reaction was allowed to proceed, and a block copolymer (1) was manufactured.

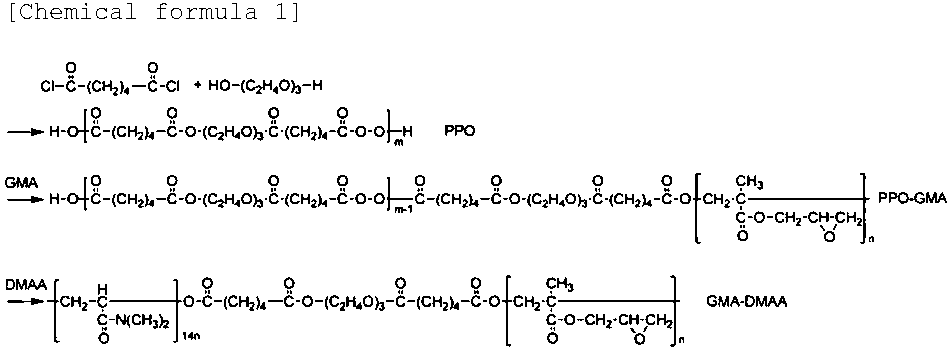

After 29.7 g of triethylene glycol was added dropwise to 72.3 g of adipic acid dichloride at 50°C, hydrochloric acid was removed at 50°C for 3 hours under reduced pressure to obtain an oligoester. Next, 4.5 g of methyl ethyl ketone was added to 22.5 g of the obtained oligoester, and the mixture was added dropwise to a solution containing 5 g of sodium hydroxide, 6.93 g of 31% hydrogen peroxide, 0.44 g of dioctyl phosphate as a surfactant, and 120 g of water and reacted at -5°C for 20 minutes. The resulting product was repeatedly washed with water and methanol, and then, dried to obtain a poly peroxide (PPO) having a plurality of peroxide groups in a molecule.

Next, 0.5 g of this PPO, 9.5 g of glycidyl methacrylate (GMA), and also 30 g of benzene as a solvent were polymerized while being stirred at 80°C for 2 hours under reduced pressure. The reactant obtained after the polymerization was reprecipitated with diethyl ether to obtain polyglycidyl methacrylate having a plurality of peroxide groups in a molecule (PPO-GMA).

Subsequently, 1.0 g of the obtained PPO-GMA (corresponding to 7 mmol of GMA) was charged into 9.0 g of N,N-dimethylacrylamide (DMAA) and 90 g of dimethyl sulfoxide as a solvent, and the mixture was reacted at 80°C for 18 hours. The reaction product obtained after the reaction was reprecipitated with hexane and recovered to obtain a block copolymer (1) (structural unit (A) : structural unit (B) = GMA : DMAA = 1 : 14 (molar ratio)) having an epoxy group in the molecule and exhibiting lubricating property in a wet state. The block copolymer (1) thus obtained was analyzed by ¹H-NMR and ATR-IR, and it was confirmed that an epoxy group was present in the molecule. In addition, The weight average molecular weight (Mw) of the block copolymer (1) measured by gel permeation chromatography (GPC, polystyrene equivalent) was about 1.5 million.

### Example 1

Polyvinyl chloride (manufactured by FUJIFILM Wako Pure Chemical Corporation, Mw = 50,000 or more) was dissolved in N,N-dimethylformamide (DMF) so that the final concentration in the coating liquid was 0.01% by mass (solution (1)). The block copolymer (1) synthesized in Synthesis Example 1 was added to and dissolved in the solution (1) so that the final concentration in the coating liquid was 4.5% by mass to prepare a coating liquid (1).

A nylon elastomer (VESTAMID E47-S1 manufactured by Evonik Industries AG) tube (outer diameter: 2.80 mm) was immersed in the coating liquid (1), and dried at room temperature (25°C) for 1 hour to remove DMF, thereby forming a coating film (1) on the tube surface (tube (1)). Further, the tube (1) was stored in an oven at 110°C for 1 hour, and the coating film (1) was heat-treated (tube (1')). The tube (1') was dried at room temperature (25°C) to produce a sample (cover tube) (1) having a cover layer (surface lubricating layer) (dry film thickness: 1 µm) including a crosslinked copolymer derived from the block copolymer (1) on the surface.

### Example 2

A sample (cover tube) (2) was produced by the same method as in Example 1 except for using polyurethane (polyester-based) (Elastollan (registered trademark) C95A manufactured by BASF Japan Ltd., Mw = 10,000 or more) in place of polyvinyl chloride in Example 1.

### Example 3

A sample (cover tube) (3) was produced by the same method as in Example 1 except for using polyurethane (polyether-based) (Elastollan (registered trademark) 1195ATR manufactured by BASF Japan Ltd., Mw = 10,000 or more) in place of polyvinyl chloride in Example 1.

### Example 4

A sample (cover tube) (4) was produced by the same method as in Example 1 except for changing the final concentration of polyvinyl chloride in the coating liquid to 0.001% by mass in Example 1.

### Example 5

A sample (cover tube) (5) was produced by the same method as in Example 1 except for changing the final concentration of polyvinyl chloride in the coating liquid to 0.005% by mass in Example 1.

### Example 6

A sample (cover tube) (6) was produced by the same method as in Example 1 except for changing the final concentration of polyvinyl chloride in the coating liquid to 0.1% by mass in Example 1.

### Example 7

A sample (cover tube) (7) was produced by the same method as in Example 1 except for changing the final concentration of polyvinyl chloride in the coating liquid to 0.5% by mass in Example 1.

### Example 8

A sample (cover tube) (8) was produced by the same method as in Example 1 except for changing the final concentration of polyvinyl chloride in the coating liquid to 2.0% by mass in Example 1.

### Example 9

A sample (cover tube) (9) was produced by the same method as in Example 1 except for changing the final concentration of polyvinyl chloride in the coating liquid to 3.0% by mass in Example 1.

### Example 10

A sample (cover tube) (10) was produced by the same method as in Example 1 except for changing the final concentration of polyvinyl chloride in the coating liquid to 4.0% by mass in Example 1.

### Example 11

A sample (cover tube) (11) was produced by the same method as in Example 1 except for changing the final concentration of polyvinyl chloride in the coating liquid to 4.5% by mass in Example 1.

### Example 12

Polyvinyl chloride (manufactured by FUJIFILM Wako Pure Chemical Corporation, Mw = 50,000 or more) was dissolved in N,N-dimethylformamide (DMF) so that the final concentration in the coating liquid was 0.1% by mass (solution (2)). The block copolymer (1) synthesized in Synthesis Example 1 was added to and dissolved in the solution (2) so that the final concentration in the coating liquid was 4.5% by mass to prepare a coating liquid (2).

A stainless steel rod (outer diameter: 1.50 mm) was immersed in the coating liquid (2), and dried at room temperature (25°C) for 1 hour to remove DMF, thereby forming a coating film (2) on the surface of the stainless steel rod (stainless steel rod (2)). Further, the stainless steel rod (2) was stored in an oven at 110°C for 12 hours, and the coating film (2) was heat-treated (stainless steel rod (2')). The stainless steel rod (2') was dried at room temperature (25°C) to produce a sample (cover stainless steel rod) (12) having a cover layer (surface lubricating layer) (dry film thickness: 0.8 µm) including the crosslinked copolymer derived from the block copolymer (1) on the surface.

### Example 13

A sample (cover stainless steel rod) (13) was produced by the same method as in Example 12 except that the final concentration of polyvinyl chloride in the coating liquid was changed to 0.5% by mass in Example 12.

### Comparative Example 1

The block copolymer (1) synthesized in Synthesis Example 1 was dissolved in N,N-dimethylformamide (DMF) so that the final concentration in the coating liquid was 4.5% by mass to prepare a comparative coating liquid (1).

A nylon elastomer (VESTAMID E47-S1 manufactured by Evonik Industries AG) tube (outer diameter: 2.80 mm) was immersed in the comparative coating liquid (1), and dried at room temperature (25°C) for 1 hour to remove DMF, thereby forming a coating film (3) on the tube surface (comparative tube (1)). Further, the comparison tube (1) was stored in an oven at 110°C for 1 hour, and the coating film (3) was heat-treated (comparison tube (1')). The comparative tube (1') was dried at room temperature (25°C) to produce a comparative sample (comparative cover tube) (1) having a cover layer (surface lubricating layer) (dry film thickness: 1 µm) including a crosslinked copolymer derived from the block copolymer (1) on the surface.

### Comparative Example 2

Polystyrene was dissolved in N,N-dimethylformamide (DMF) so that the final concentration in the coating liquid was 0.1% by mass (comparative solution (2)). The block copolymer (1) synthesized in Synthesis Example 1 was added to and dissolved in the comparative solution (2) so that the final concentration in the coating liquid was 4.5% by mass to prepare a comparative coating liquid (2).

A nylon elastomer (VESTAMID E47-S1 manufactured by Evonik Industries AG) tube (outer diameter: 2.80 mm) was immersed in the comparative coating liquid (2), and dried at room temperature (25°C) for 1 hour to remove DMF, thereby forming a coating film (4) on the tube surface (comparative tube (2)). Further, the comparison tube (2) was stored in an oven at 110°C for 1 hour, and the coating film (4) was heat-treated (comparison tube (2')). The comparative tube (2') was dried at room temperature (25°C) to produce a comparative sample (comparative cover tube) (2) having a cover layer (surface lubricating layer) (dry film thickness: 1 µm) including a crosslinked copolymer derived from the block copolymer (1) on the surface.

### Comparative Example 3

The block copolymer (1) synthesized in Synthesis Example 1 was dissolved in N,N-dimethylformamide (DMF) so that the final concentration in the coating liquid was 4.5% by mass to prepare a comparative coating liquid (3).

A stainless steel rod (outer diameter: 1.50 mm) was immersed in the comparative coating liquid (3), and dried at room temperature (25°C) for 1 hour to remove DMF, thereby forming a coating film (5) on the surface of the stainless steel rod (comparative stainless steel rod (3)). Further, the comparative stainless steel rod (3) was stored in an oven at 110°C for 12 hours, and the coating film (5) was heat-treated (comparative stainless steel rod (3')). The comparative stainless steel rod (3') was dried at room temperature (25°C) to produce a comparative sample (cover stainless steel rod) (3) having a cover layer (surface lubricating layer) (dry film thickness: 0.8 µm) including a crosslinked copolymer derived from the block copolymer (1) on the surface.

The sliding durability and sliding property of each of the samples (1) to (13) produced in Example 1 to 13 and the comparative samples (1) to (3) produced in Comparative Examples 1 to 3 were evaluated according to the following methods. The results are shown in Table 1 below. In the following Table 1, the block copolymer (1) synthesized in the above Synthesis Example 1 is represented as "p(GMA-b-DMAA)". The mixing ratio of the polymer material to 100 parts by mass of the block copolymer is referred to as "polymer material/100 part block copolymer".

### [Evaluation of sliding durability]

For each sample, sliding durability (durability of the surface lubricating layer) was evaluated according to the following method.

Each sample was immerse in tap water, and a sensory evaluation of sliding durability was performed by a method in which the sample was grasped by hand and rubbed. The sliding durability was evaluated by measuring the number of times of rubbing until the lubricating property (smoothness) was impaired.

It is determined that the sliding durability is more excellent as the number of times is larger. The upper limit number of rubs was set to 50, and the sample in which the sliding property was retained at the time of 50 rubs was described as >50.

### [Evaluation of sliding property]

For each sample, sliding property (sliding property of the surface lubricating layer) was evaluated according to the following method.

Each sample was immerse in tap water, and a sensory evaluation of a sliding property was performed by a method in which the sample was grasped by hand and rubbed according to the following evaluation criteria.

### (Evaluation criteria)

⊙: The film is rubbed 50 times, the coated state is not changed before and after rubbing, and the sliding property is also excellent;
∘: the coated state is not changed before and after rubbing, but the sliding property is slightly poor;
△: the coated state is changed by rubbing (roughness, etc.), but a constant sliding property is retained even after 50 times rubbing; and
×: the coating state is changed by rubbing, and the coating is peeled off partially or entirely in the evaluation section after 50 times rubbing.

### [Table 1]

**Table 1**

| | Base layer | Polymeric material | | Solvent | Block copolymer | | Polymeric material/100 part block copolymer | Conditions of heat treatment | Sliding durability | Sliding property |
|---|---|---|---|---|---|---|---|---|---|---|
| | | Type | Mass ratio (to solvent) | | Type | Mass ratio (to solvent) | | | | |
| Example 1 | Nylon elastomer | Polyvinyl chloride | 0.01 | DMF | p (GMA-b-DMAA) | 45 | 0.22 | 110°C × 1 hr | > 50 | ⊙ |
| Example 2 | Nylon elastomer | Polyurethane (polyester-based) | 0.01 | DMF | p (GMA-b-DMAA) | 4.5 | 0.22 | 110°C × 1 hr | > 50 | ⊙ |
| Example 3 | Nylon elastomer | Polyurethane (polyester-based) | 0.01 | DMF | p (GMA-b-DMAA) | 4.5 | 0.22 | 110°C × 1 hr | > 50 | ⊙ |
| Example 4 | Nylon elastomer | Polyvinyl chloride | 0.001 | DMF | p (GMA-b-DMAA) | 4.5 | 0.02 | 110°C × 1 hr | > 50 | Δ |
| Example 5 | Nylon elastomer | Polyvinyl chloride | 0.005 | DMF | p (GMA-b-DMAA) | 4.5 | 0.11 | 110°C × 1 hr | > 50 | ⊙ |
| Example 6 | Nylon elastomer | Polyvinyl chloride | 0.1 | DMF | p (GMA-b-DMAA) | 4.5 | 2.22 | 110°C × 1 hr | > 50 | ⊙ |
| Example 7 | Nylon elastomer | Polyvinyl chloride | 0.5 | DMF | p (GMA-b-DMAA) | 45 | 11.11 | 110°C × 1 hr | > 50 | ⊙ |
| Example 8 | Nylon elastomer | Polyvinyl chloride | 2.0 | DMF | p (GMA-b-DMAA) | 45 | 4444 | 110°C × 1 hr | > 50 | ⊙ |
| Example 9 | Nylon elastomer | Polyvinyl chloride | 3.0 | DMF | p (GMA-b-DMAA) | 4.5 | 66.67 | 110°C × 1 hr | > 50 | ⊙ |
| Example 10 | Nylon elastomer | Polyvinyl chloride | 40 | DMF | p (GMA-b-DMAA) | 4.5 | 88.89 | 110°C × 1 hr | > 50 | ⊙ |
| Example 11 | Nylon elastomer | Polyvinyl chloride | 4.5 | DMF | p (GMA-b-DMAA) | 45 | 100.00 | 110°C × 1 hr | > 50 | ○ |
| Example 12 | Stainless steel | Polyvinyl chloride | 0.1 | DMF | p (GMA-b-DMAA) | 4.5 | 2.22 | 110°C × 12 hr | > 50 | ⊙ |
| Example 13 | Stainless steel | Polyvinyl chloride | 05 | DMF | p (GMA-b-DMAA) | 45 | 11.11 | 110°C × 12 hr | > 50 | Δ |
| Comparative Example 1 | Nylon elastomer | None | - | DMF | p (GMA-b-DMAA) | 4.5 | - | 110°C × 1 hr | 18 | × |
| Comparative Example 2 | Nylon elastomer | Polystyrene | 0.1 | DMF | p (GMA-b-DMAA) | 4.5 | 2.22 | 110°C × 1 hr | 7 | × |
| Comparative Example 3 | Stainless steel | None | - | DMF | p (GMA-b-DMAA) | 4.5 | - | 110°C × 12 hr | 25 | × |

Table 1 shows that a medical device having a surface lubricating layer excellent in sliding durability and the sliding property can be obtained by applying a coating liquid including polyvinyl chloride or polyurethane onto a base layer.

On the other hand, when polyvinyl chloride or polyurethane was not used for the coating liquid, the surface lubricating layer had poor sliding durability and a poor sliding property (Comparative Example 1 and Comparative Example 3). This is presumed to be because crosslinking or polymerization of the block copolymer was not promoted, and a strong surface lubricating layer was not formed. When polystyrene was used as a coating liquid, the surface lubricating layer had poor sliding durability and a poor sliding property (Comparative Example 2). It is presumed that this is because since polystyrene has no electron-withdrawing property, crosslinking or polymerization of the block copolymer was not promoted, and a strong surface lubricating layer was not formed.

Comparison between Examples 1 and 4 to 11 and Comparative Example shows that when the addition amount of the polymer material is 0.001% by mass or more, sufficient durability (sliding durability) can be exhibited. When the addition amount of the polymer material was 0.005 to 4.0% by mass, a more excellent sliding property was exhibited. From this, it is considered that the addition amount of the polymer material is preferably 0.005 to 4.0% by mass from the viewpoint of the sliding property.

Next, the sliding resistance of each of the samples (12) to (13) produced in Examples 12 to 13 and the comparative sample (3) produced in Comparative Example 3 was evaluated according to the following method. Results thereof are presented in Table 2 below. In the following Table 2, the block copolymer (1) synthesized in the above Synthesis Example 1 is represented as "p(GMA-b-DMAA)". The mixing ratio of the polymer material to 100 parts by mass of the block copolymer is referred to as "polymer material/100 part block copolymer".

### [Evaluation of sliding resistance]

Each sample was set in a pinch tester (DL1000 manufactured by OAKRIVER TECHNOLOGY) in a state of being immersed in tap water, and was slid 100 times at a grip force of 500gf, a test speed of 8.3 mm/s, and a test stroke of 25 mm (grip pad material: silicone, grip pad height: 12.35 mm). The sliding property was evaluated by measuring the test force at the time of sliding 100 times. It is determined that the lower the test force is, the more excellent the sliding durability and the sliding property are.

### [Table 2]

**Table 2**

| | Base layer | Polymeric material | | Solvent | Block copolymer | | Polymeric material/100 part block copolymer | Conditions of heat treatment | Test force (gf) |
|---|---|---|---|---|---|---|---|---|---|
| | | Type | Mass ratio (to solvent) | | Type | Mass ratio (to solvent) | | | |
| Example 12 | Stainless steel | Polyvinyl chloride | 0.1 | DMF | p (GMA-b-DMAA) | 4.5 | 2.22 | 110°C × 12 hr | 12 |
| Example 13 | Stainless steel | Polyvinyl chloride | 0.5 | DMF | p (GMA-b-DMAA) | 4.5 | 11.11 | 110°C × 12 hr | 79 |
| Comparative Example 3 | Stainless steel | None | - | DMF | p (GMA-b-DMAA) | 4.5 | - | 110°C × 12 hr | 243 |

Table 2 shows that a medical device having a surface lubricating layer excellent in sliding durability and sliding property can be obtained by applying a coating liquid including polyvinyl chloride onto a base layer.

On the other hand, when polyvinyl chloride was not used for the coating liquid, the sliding property of the surface lubricating layer was poor (Comparative Example 3). This is considered to be because crosslinking or polymerization of the block copolymer was not promoted, and a strong lubricating layer was not formed, so that a part of the surface lubricating layer was peeled off at the time of sliding 100 times.

In addition, when comparing Examples 12 and 13, in the case where the addition amount of polyvinyl chloride was 0.1% by mass (Example 12), the more excellent sliding property was exhibited as compared with the case where the addition amount was 0.5% by mass (Example 13). This is considered to be because crosslinking or polymerization was further promoted by an increase in the amount of polyvinyl chloride to be added, and thus sliding property was deteriorated.

The present application is based on Japanese Patent Application No. 2023-037345 filed on March 10, 2023, the entire content of which is incorporated herein by reference.

### Reference Signs List

- 10: Medical device
- 1: Base layer
- 1a: Base layer core portion
- 1b: Base material surface layer
- 2: Lubricating layer

## Claims

1. A method for manufacturing a medical device including a base layer and a surface lubricating layer supported on at least a part of the base layer, the method comprising:
preparing a coating liquid including a block copolymer having a structural unit (A) derived from a reactive monomer having an epoxy group and a structural unit (B) derived from a hydrophilic monomer, at least one polymer material selected from polyvinyl chloride and polyurethane, and a solvent; and
applying the coating liquid onto the base layer.

2. The method according to claim 1, wherein a content of the polymer material in the coating liquid is smaller than a content of the block copolymer in the coating liquid.

3. The method according to claim 1, wherein the coating liquid comprises the polymer material in an amount of 0.001% by mass or more and less than 12% by mass.

4. The method according to claim 1, wherein the reactive monomer having an epoxy group comprises at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.

5. The method according to claim 1, wherein the hydrophilic monomer comprises at least one selected from the group consisting of N,N-dimethylacrylamide, acrylamide, 2-hydroxyethyl methacrylate, and N-vinylpyrrolidone.

6. A medical device comprising: a base layer; and a surface lubricating layer supported on at least a part of the base layer, wherein
the surface lubricating layer includes a block copolymer having a structural unit (A) derived from a reactive monomer having an epoxy group and a structural unit (B) derived from a hydrophilic monomer, and at least one polymer material selected from polyvinyl chloride and polyurethane, and
in the surface lubricating layer, the block copolymer and the polymer material are interpenetrated with each other.

7. The medical device according to claim 6, wherein an amount of the polymer material present in the surface lubricating layer is smaller than an amount of the block copolymer present in the surface lubricating layer.

8. The medical device according to claim 6, wherein the reactive monomer having an epoxy group comprises at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.

9. The medical device according to claim 6, wherein the hydrophilic monomer comprises at least one selected from the group consisting of N,N-dimethylacrylamide, acrylamide, 2-hydroxyethyl methacrylate, and N-vinylpyrrolidone.
